(19) **Europäisches Patentamt / European Patent Office / Office européen des brevets**

(11) **EP 2 301 495 A1**

(12) # EUROPEAN PATENT APPLICATION

(43) Date of publication:
30.03.2011 Bulletin 2011/13

(51) Int Cl.:
*A61F 13/02* (2006.01)

(21) Application number: 10251650.7

(22) Date of filing: 24.09.2010

(84) Designated Contracting States:
AL AT BE BG CH CY CZ DE DK EE ES FI FR GB GR HR HU IE IS IT LI LT LU LV MC MK MT NL NO PL PT RO SE SI SK SM TR
Designated Extension States:
BA ME RS

(30) Priority: 25.09.2009 US 566941

(71) Applicant: Johnson & Johnson Consumer Companies, Inc.
Skillman, NJ 08558 (US)

(72) Inventor: Da Silva Macedo Jr., Carlos
Sao Jose dos Campos
SP 12233002 (BR)

(74) Representative: Carpmaels & Ransford
One Southampton Row
London WC1B 5HA (GB)

(54) **Cushioned adhesive bandage**

(57) The present invention relates to an adhesive bandage for use on wounds placed under compressive force, which includes a support layer having a top side and an opposing bottom side, an adhesive layer deposited onto the bottom side of the support layer, a substantially sealed cushion pad associated with the adhesive layer, and an absorbent pad bonded to the cushion pad.

**Fig. 2**

EP 2 301 495 A1

**Description**

**FIELD OF THE INVENTION**

The present invention relates to an adhesive bandage to be applied onto the skin, particularly an adhesive bandage for use on skin wounds that are subjected to compressive force.

**BACKGROUND OF THE INVENTION**

There are many types of wounds to the human body. They may be open or closed. Open wounds include incisions or incised wounds, lacerations, abrasions (grazes), puncture wounds, penetration wounds and gunshot wounds. Closed wounds include contusions (bruises), hematomas and crushing injuries.

Adhesive bandages intended for use on the skin, protecting the skin from injuries or the wound(s) from dirt and guaranteeing the efficacy of medicaments applied topically, typically include at least one film made of a liquid-impermeable material that also prevents contamination of the wound.

Conventionally, at least one pad comprising at least one absorbent material and at least one adhesive segment is associated to this film in order to enable fixation of the adhesive bandage to the user's skin. Preferably, the adhesive element is positioned on the adhesive bandage in such a manner that the region intended to be in contact with the wound (the pad) does not have adhesive, since the presence thereof could entail maceration at the wound upon removal or replacement of the adhesive bandage.

In order to accelerate the healing of the wound and enable skin respiration, the film is permeable to gas and may further contain a plurality of through bores.

With a view to increase more and more the efficacy and the comfort provided by the adhesive bandage, a number of improvements in this extremely efficient basic concept were developed, as for instance, the improvement of the materials used or the form of alterations in the coloration of the adhesive bandage.

In some cases, adhesive bandages are used where they are under a compressive force. For example, if a person has a wound under an article of clothing, the clothing will exert force on the bandage and wound, thus compressing the adhesive bandage.

Such is the case when a person has a blister. Blisters are small swellings of the skin that contains watery fluid. They are caused by friction from shoes or clothing which rubs repeatedly on the skin, thus causing friction bums. The body responds to the friction by producing fluid. The fluid builds up beneath the part of the skin being rubbed, causing pressure and pain.

Blisters are best treated by allowing them to be exposed to air, so that the area can dry and the blister be reabsorbed into the skin. Other wounds are often covered with adhesive bandages, protecting the wound(s) from dirt or contamination. In the case of blisters, most people do not have the time to wait for healing to occur and must wear clothing or shoes over the blister. To treat a blister when clothing or shoes are worn over it, a user often covers the blister with an adhesive bandage. The pad absorbs any fluid leaking from the blister and provides a cushion between the blister and the surface of the shoe or sock.

To avoid blisters, people "breaking in" new shoes will often wear athletic tape or adhesive bandages on their feet at the locations where the new shoes exert friction and pressure on the foot. There are several problems with these practices. Athletic tape prevents friction, but does not address the issue of cushioning the blister. Adhesive bandages with pads cushions the area, but the pad deforms under the pressure of the clothing or shoes, minimizing its ability to cushion the area.

In summary, adhesive bandages providing additional cushioning to wounds or areas of the skin that are subjected to compressive forces are needed.

**SUMMARY OF THE INVENTION**

The present invention relates to an adhesive bandage for use on wounds placed under compressive force, which includes a support layer having a top side and an opposing bottom side, an adhesive layer deposited onto the bottom side of the support layer, a substantially sealed cushion pad associated with the adhesive layer, and an absorbent pad bonded to the cushion pad.

**BRIEF DESCRIPTION OF THE DRAWINGS**

The present invention will now be described in greater detail with reference to embodiments represented in the drawings.

FIG. 1 is a perspective view of a first embodiment of the adhesive bandage of the present invention;

FIG. 2 is a cross-sectional view of the adhesive bandage illustrated in FIG. 1 taken along plane A---A;

FIG. 3 is a cross-sectional side view of a cushion pad embodiment of the adhesive bandage illustrated in FIG. taken along plane A---A;

FIG. 4a is a cross-sectional side view of a second cushion pad embodiment of the adhesive bandage of the present invention;

FIG. 4b is a top sectional view along plane C -- C of the second cushion pad embodiment illustrated in FIG. 4a; and

FIG. 5 is a cross-sectional side view of a second embodiment of the adhesive bandage of the present invention

## DETAILED DESCRIPTION OF THE INVENTION

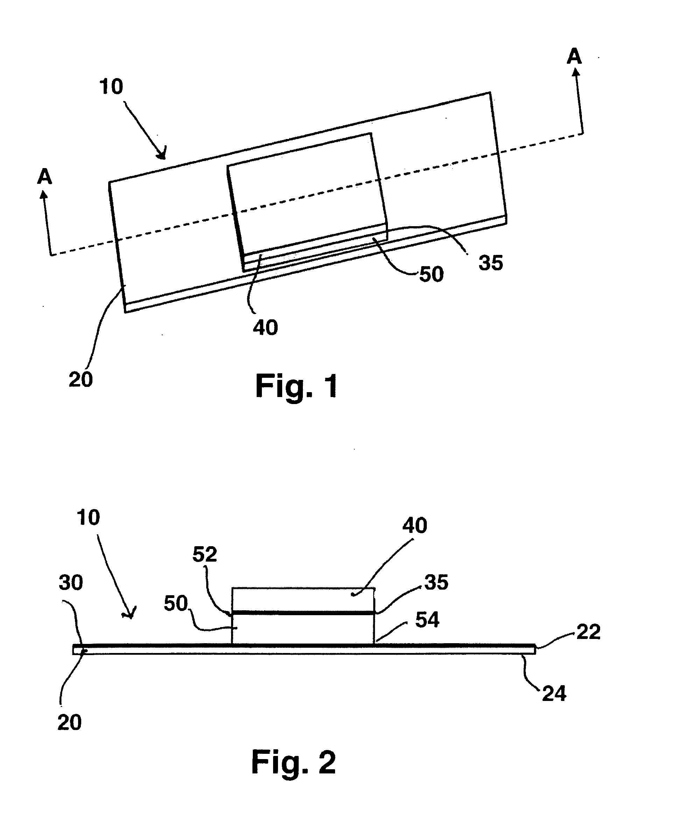

FIGS. 1-3 illustrate a first embodiment of the adhesive bandage of the present invention. The adhesive bandage **10** comprises at least one support layer **20** having top side **24** and opposing bottom side **22**. An adhesive layer **30** is associated to bottom side **22** of support layer **20**. Top side **54** of cushion pad **50** is associated with adhesive layer **30**. Bottom side **52** of cushion pad **50** is bonded to absorbent pad **40** via bonding layer **35**. Bonding may be achieved by use of an adhesive, or by any other known means of bonding, such as by ultrasonic welding.

The support layer may have various shapes, e.g. rectangular, circular, oblong, etc. The shape of adhesive bandage **10** is defined by the shape of support layer **20**. The composition of the support layer may vary, but it is preferably made from a polyolefin, polyurethane polymer, polyethylene, vinyl polyethylene acetate, polyurethane foam film, and may further be made from a textile non-woven fabric, rubber, or other materials known in the adhesive bandage arena.

By preference, polymers used to make the support layer exhibit viscosity of about 500 to 500,000 centipoises at temperatures lower than 190°C, or about 1,000 to 30,000 centipoises at temperatures lower than 190°C, or about 3,000 to 15,000 centipoises at temperatures lower than 190°C.

The support layer may be impermeable to liquid, but permeable to gas, which allows the wound and the skin to which the adhesive bandage **10** is adhered to breath. For this, the support layer has pores of such a size that will allow only the passage of gases, which have molecules of extremely small size.

Finally, one can conceive a support layer that is perforated for more ventilation of the skin. However, the support layer may still be totally impermeable to gases, when necessary.

Adhesive layer **30** is associated with support layer **20**. Bonding layer **35** may be in the form of an adhesive, and is associated with top side **54** of cushion pad **50**. Adhesive layer **30** comprises an adhesive, so that it will be able to stick to the user's skin. Bonding layer **35** may comprise an adhesive to bond cushion pad **50** to absorbent pad **40**.

In general, any of a variety of pressure-sensitive adhesives can be utilized as the adhesive layer and the bonding layer in the present invention. In particular, pressure-sensitive adhesives that are biocompatible with human skin are typically utilized. Moreover, an adhesive of the present invention is also either generally water soluble or generally insoluble, or dispersible in an aqueous environment. For instance, one preferred, commercially available dispersible pressure-sensitive adhesive is sold under the trade name of HL-9415-X and is available from H.B. Fuller Company. Another suitable adhesive includes about 10-75% by weight of a polyalkyloxazoline polymer, 10-75% by weight of a functional diluent comprising a hydroxy compound or a carboxylic acid compound, and 5-50% by weight of a tackifier.

The water-dispersible polymeric component can include, for example, surfactants such as poly(ethylene oxide) alkylphenyl ethers, such as those sold under the trade names IGEPAL.CO and IGEPAL.CA (available from Rhone-Poulenc, Inc.); poly(ethylene oxide) lauryl, cetyl, and oleyl ethers such as those sold under the trade name BRIJ (available from ICI Americas, Inc.); poly(ethylene oxide) laurate; poly(ethylene oxide) oleate; sorbitan oleate; ethylene oxide/propylene oxide block copolymers such as those sold under the trade names PLURONIC and TETRONIC (available from BASF Corporation); and organic phosphate esters, such as those sold under the trade name GAFAC PE-510 (available from International Specialty Products). Examples of other components include, but are not limited to, poly(acrylic acid); poly(vinyl alcohol); poly(N-vinyl pyrrolidone); poly(acrylamide); poly(alkoxyalkyl (meth)acrylates), such as 2-ethoxy ethyl acrylate, 2-ethoxy ethyl methacrylate, 2-(2-ethoxyethoxy) ethyl acrylate, and 2-methoxy ethyl acrylate (available from SARTOMER Company, Inc.); poly(vinyl methyl ether); poly(vinyl methyl ether: maleic anhydride), sold under the trade name GANTREZ (available from International Specialty Products); poly(ether polyols), such as poly(propylene glycol) and the like, such as those sold under the trade name SANNIX (available from Sanyo Chemical Industries); copolymers thereof, and the like. Copolymers of these and alkyl (meth)acrylate esters or vinyl esters are also suitable. Gums such as those derived from okra and guar may also be used.

Still another suitable pressure-sensitive adhesive includes about 10% to about 80%, by weight, of an alkali soluble polymer; about 0 to about 30%, by weight, of a poly(vinyl methyl ether); about 30% to about 70%, by weight, of a tackifying resin; and about 5% to about 30%, by weight, of a suitable plasticizer. Still other examples of suitable adhesives include HX 9236-01 or HX 9237-01 hot melt adhesives, which are obtainable from ATO Findley, Inc.

The adhesive layer and bonding layer may comprise hydrocolloids. The hydrocolloid element used may be any substance that has a good performance in this utilization, as for example, sodium carboxymethylcellulose, pectin, xanthan gum, polysaccharides, sodium or calcium alginates, chitosan, seaweed extract (cageenan), polyaspartic acid, polyglutamic acid, hyaluronic acid or salts and derivatives thereof, among others.

Hydrocolloids, just as sodium carboxymethylcellulose and pectin, among others, are agents that form gels as soon as they come into contact with the bodily fluids from the wound. When used in adhesive bandages, these hydrocolloids are combined with elastomers and/or adhesives. Preferably, the adhesive bandage should guarantee a humid environment but without saturation, cicatrisation, which is a situation suitable for acceleration of the healing,

Pectin is a complex-structure polysaccharide, extracted from vegetable species (as for example, peels from citric fruits or apple pulp), which has a highly hydrophilic structure and, as a result, associate easily with the water molecules of the bodily fluids from the wound, forming a viscous gel on the injury bed. Its chemical similarity with alginates causes the physical properties of absorption and gel formation to resemble each other.

Carboxymethylcellulose, in turn, is a cellulose derivative, formed by reaction of cellulose with alkalis (such as, for example, sodium, potassium, calcium, etc., hydroxide). It is the nature of combined alkali that imparts the ionic characteristic of carboxymethylcellulose (when sodium hydroxide is used, sodium carboxymethylcellulose is formed). Just as in the case of pectin, carboxymethylcellulose dissolves rapidly in the water coming from the liquids that emanate from the wound, forming a gel on the wound with controlled viscosity.

As an additional advantage of the use of hydrocolloids, it should be noted that both pectin and carboxymethylcellulose form a gel with acidic characteristics (pH of about 4), functioning as a bactericidal agent.

Before the use of adhesive the bandage, the hydrocolloid is substantially inert to water vapor; but, as soon as the gelling process begins, the adhesive bandage becomes progressively more permeable. The gelling process continues, provided that the injury continues to exude bodily fluids, until the whole hydrocolloid is used up, at which time saturation of the adhesive bandage is reached, and it should be replaced.

The adhesive element used may be any conventional adhesive know for such use, as for example pressure acrylic adhesives, among others. Additionally, such an adhesive may contain a resin for increasing adhesion, a cohesion increasing agent, an absorption agent (preferably a polyacrylate superabsorbent, a polyacrylate salt superabsorbent or a mixture thereof), a plasticizer and optionally a pigment. The adhesive layer may further be configured in discontinuous patterns, arranged in lines, screen, spray or any other which a person skilled in the art understands as discontinuous, composed by an elastomeric base. In some embodiments, the adhesive layer and the bonding layer are comprised of the same material.

Over the adhesive layer is positioned at least one cushion pad. The cushion pad is sized to cover less area than the support layer, so that in use the adhesive layer is in contact with the user's skin, but preferably does not contact the wound surface.

Over the cushion pad is positioned at least one absorbent pad, which substantially covers the wound area and is in contact with the wound surface. The absorbent pad is sized to cover less area than, or no more than up to, the area of the cushion pad. The bonding layer bonds the absorbent pad to the top side of the cushion pad.

The absorbent pad can be made from any type of material commonly used in the art in forming such pads. The absorbent pad can be made from cellulose fiber or non-biodegradable or biodegradable foam. Also, the absorbent pad may be wrapped or laminated with a cover of plastic or treated cellulose to prevent bonding to the wound tissue.

In some embodiments, the absorbent pad can also be dispersible in water to facilitate disposal of the adhesive bandage. Water-dispersible foam composites are one example of a suitable absorbent pad that can be used in the present invention. Other suitable materials that can be used for the absorbent pad include lightly cross-linked tissue structures, absorbent films, and the like.

In some embodiments, the absorbent pad can comprise hydrocolloids such as those described above. In these embodiments, the absorbent pad is in the form of an absorbent adhesive layer, and will directly bond with the cushion pad, thus serving the dual function of the bonding layer and the absorbent pad. The adhesiveness of the absorbent pad is controlled by the adhesiveness of above listed elastomers and adhesives, and the ratio of hydrocolloid to elastomers and/or adhesives in the absorbent pad.

The absorbent pad may comprise discontinuities selected from the group consisting of pores, slots, perforations, cracks, grooves, openings, holes or the like. The absorbent pads may comprise an open area, referring to the sum of the area of discontinuities within the absorbent pad, of about 10% to 90%, or of about 25% to 60% or still of about 30 to 40%. The pore area may be about 0.001 to 10 mm$^2$, or from 0.1 to 1 mm$^2$, or about 0.1 to 0.5 mm$^2$.

The cushion pad provides cushioning to help protect a wound or blister, as well as to provide comfort for the user, specifically for wounds or blisters placed under compressive force. The cushion pad comprises a material that will deform when subjected to a compressive stress, but will recover its original shape when the stress is released. The cushion pad may be in the form of a foam comprised of polymers such as polystyrene, polyolefin, rigid polyurethane, flexible polyurethane, polystyrene and polyvinyl chloride. The foams have closed cell pores due to sealing of the cushion pads, usually filled with air. The volume of pores comprise about 80% to about 99%, or about 90% to about 97%, of the volume of the foam. Typical average pore diameter is about 0.1mm to about 1.0mm. One skilled in the art, after having the benefit of this disclosure will be able to ascertain many combinations of material (polymer chemistry, molecular weight), porosity and pore size that may be used to achieve the performance required for the cushion pad.

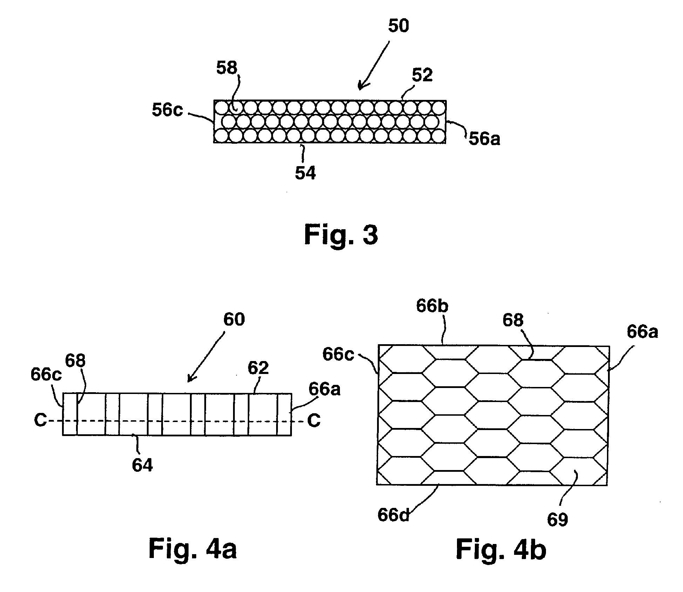

FIG. 3 illustrates a first embodiment of a cushion pad **50** of the present invention. The figure is a sectional schematic view along plane A---A of the adhesive bandage illustrated in FIG. 1, showing only cushion pad **50**. Cushion pad 50 has top **54**, bottom **52**, sides **56a** and **56c**, and closed cells in the form of closed pores **58**. Though shown in the figure as three lines of uniform-sized closed pores **58** through the thickness of cushion pad **50**, closed pores **58** may be of uniform or non-uniform size, and may be randomly or non-randomly dispersed through cushion pad **50**.

Top side **54**, bottom side **52** and sides **56a** and **56c** of cushion pad **50** are substantially sealed, so as to provide closed pores, or cells within the body of the cushion pad. By substantially sealed, it is meant that the perimeter of the cushion pad is sealed or otherwise treated, so as to prevent air contained within pores or cells within the body of the cushion pad to be forced from the cushion pad upon subjection to compressive forces, to the extent that the average percent thickness recovery of such substantially sealed cushion pad is at least 80% when subjected to a force of 8 Newtons (N) for five minutes.

FIG. **4a** illustrates a cross-sectional side view a second embodiment of a cushion pad of the present invention. Cushion pad **60** has top **64**, bottom **62**, sides **66a** and **66c**, and internal wall structure **68**.

FIG. 4b is a top cross-sectional view along plane C---C of the second cushion pad **60** embodiment illustrated in FIG. 4a. The figure shows sides **66a** through **66d**, and internal wall structure **68** of cushion pad **60**. Cushion pad **60** is generally in the form of a sandwich structured composite with closed cells formed by top **64**, bottom **62**, sides **66**, and internal wall structure **68**. In the presented embodiment, wall structure **68** is in the form of a honeycomb with closed cells **69**. However, closed cells **69** may be of a different cross-section, such as square, rectangular, polygonal, oval, circular, etc.

The process of manufacturing the adhesive bandage **10** of the present invention may be any of those conventionally known to produce adhesive bandages. Support layer **20**, cushion pad **50**, absorbent pad **40**, and first adhesive layer **30** can be obtained by any methods available at present. For example, an extrusion process may be used for obtaining support layer **20**. In the same way, the adhesive layers **30** and **35** can be made in any known manner. A support layer as described herein is obtained and an adhesive layer as described herein is applied to the bottom surface of the support layer. The cushion pad is then associated to the adhesive layer. A bonding layer is then applied to the bottom side of the cushion pad. The absorbent pad is then associated with the bonding layer, thus bonding the absorbent pad to the cushion pad.

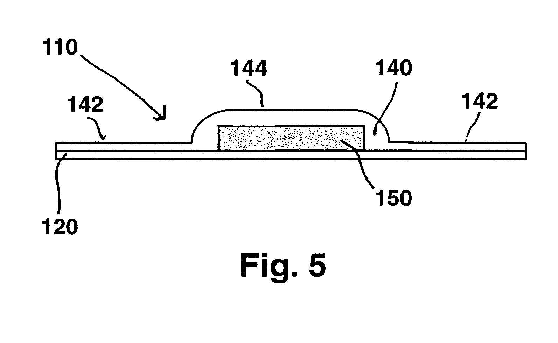

FIG. 5 is a cross-sectional side view of a second embodiment of the adhesive bandage of the present invention. Adhesive bandage **110** comprises at least one support layer **120**, to which at least one absorbent adhesive layer **140** is associated. Cushion pad **150** is associated with support layer **120**, and cushion pad **150** is covered with absorbent adhesive layer **140**.

Support layer **120** and cushion pad **150** have been described earlier. In this embodiment, absorbent adhesive layer **140** comprises a hydrocolloid, and thus serves the dual function of absorbent and adhesive layers. As discussed above, hydrocolloids include, for example, sodium carboxymethylcellulose, pectin, xanthan gum, polysaccharides, sodium or calcium alginates, chitosan, seaweed extract (carrageenan), polyaspartic acid, polyglutamic acid, hyaluronic acid or salts and derivatives thereof, among others.

As mentioned above, the hydrocolloids are blended with elastomers and/or adhesives to form absorbent adhesive layer **140**. The adhesiveness of absorbent adhesive layer **140** is controlled by the adhesiveness of above listed elastomers and adhesives, and the ratio of hydrocolloid to elastomers and/or adhesives in absorbent adhesive layer **140**.

In one embodiment, adhesive bandage **110** may have high adhesiveness to the skin in the peripheral region **142** (peripheral to cushion pad **150**), combined with less adhesiveness in the pad region **144**, thus combining healing capacity with comfort of the user at the time of replacement.

The adhesiveness in the pad region **144** may be 10% less than the adhesiveness in the peripheral region **142**. For example, the adhesiveness value in the pad region **144** is may be at least $36N/cm^2$ and the adhesiveness value in the peripheral region **142** may be at least $40 \ N/cm^2$. More preferably, the adhesiveness value in the pad region **144** is of at least $25 \ N/cm^2$ and the adhesiveness value in the peripheral region **142** is of at least $36 \ N/cm^2$. Still more preferably, the adhesiveness value in the pad region **144** is preferably of at least $10 \ N/cm^2$ and the adhesiveness value in the peripheral region **142** is of at least $25 \ N/cm^2$. Other preferred adhesiveness values in the pad region **144** are $12 \ N/cm^2$, $8 \ N/cm^2$ and $4 \ N/cm^2$, and adhesiveness values in the peripheral region **142** are $15 \ N/cm^2$, $10/cm^2$ and $8 \ N/cm^2$, respectively. Still preferably, other adhesiveness values in the pad region **144** may be, at the most, $10 \ N/cm^2$, preferably $6 \ N/cm^2$ and more preferably $1 \ N/cm^2$.

The process of manufacturing the adhesive bandage **110** of the present invention comprises the steps of obtaining support layer **120** as defined before; obtaining absorbent adhesive layer **140**; obtaining cushion pad **150**; associating cushion pad **150** and support layer **120**; and associating adhesive layer **140** with support layer **120** and cushion pad **150**.

The adhesive bandages of the invention are ideally suited to deliver one or more active ingredients such as therapeutics to the surface of the skin. Illustrative classes of active ingredients that may be delivered to the skin via the adhesive bandages of the invention include, but are not limited to, antibiotics, analgesics, antipyretics, antimicrobials, antiseptics, antiallergics, anti-acne, anesthetics, anti-inflammatories, hemostats, cosmetics, vitamins, vasodilators, emollients, pH regulators, antipruritics, counterirritants, antihistamines and steroids. Specific active ingredients that may be delivered to the skin via the dressings of the invention include chlorhexidine, neomycin sulfate, polymyxin-B sulfate, zinc bacitracin, benzalkonium chloride, cetylpyridinium chloride, bupivacaine, tetracaine, cincaine, lidocaine, benzocaine, silver sulfadiazine, hydrocortisone, metandienone, trypsin, tolazoline, heparin, pramoxine, aloe vera, tretinoin, retinol, retinaldehyde, menthol, capsaicin, alpha hydroxy acids and vitamins such as Vitamin E.

When contained in the adhesive bandages of the invention, one or more active ingredients may be contained primarily or exclusively in the absorbent pad **40** of the adhesive bandage or primarily or exclusively in the absorbent adhesive layer **140.**

Various embodiments of the invention have been set forth above. Each embodiment is provided by way of explanation of the invention, not limitation of the invention. In fact, it will be apparent to those skilled in the art that various modifications and variations can be made in the present invention without departing from the scope or spirit of the invention. For instance, features illustrated or described as part of one embodiment, can be used on another embodiment to yield a still further embodiment. Thus, it is intended that the present invention cover such modifications and variations as come within the scope of the appended claims and their equivalents.

The present invention may be better understood with reference to the following example.

**EXAMPLE 1: Thickness recovery of closed cell versus open cell pads.**

**Materials and equipments**

- Laser micrometer
- Universal tensilometer
- PMMA support plates
- Metallic plunger with a rubber tip with approximate dimensions of 6 mm length, by 11mm wide, by 10 mm thick
- Samples were cut to approximate dimensions of 6 mm length, by 20 mm width, by 2.5 mm (or 5.0 mm) thick

**Methodology**

- The thickness of the PMMA plate support was measured with the laser micrometer.
- A sample was pasted on the PMMA surface.
- The initial thickness of the sample ($t_{before}$) was measured with the laser micrometer
- The plate with sample was placed in the tensilometer base.
- The tensilometer was activated (at 30.00 mm/min) to put an 8N force on the sample.

The 8N force was maintained on the sample for 5 minutes, and then released.

- The thickness of the sample ($t_{after}$) was measured with the laser micrometer 50 seconds after compression was released.
- The thickness recovery (%REC) of the sample was determined by the equation:

$$\%REC = \frac{t_{after}}{t_{before}} \times 100$$

**Results:**

Tables 1 and 2 show the thickness recovery for sealed cushion pads (closed cell) versus unsealed cushion pads (open cell).

Table 1- Results for adhesive bandage with closed cell cushion pad.

| Measurements | Thickness (mm) | | Thickness recovery (%) |
|---|---|---|---|
| | Before pressure | After pressure | |
| 1 | 2.652 | 2.314 | 87.255 |
| 2 | 2.581 | 2.150 | 83.301 |
| 3 | 2.413 | 2.040 | 84.542 |
| 4 | 2.483 | 2.050 | 82.561 |
| 5 | 2.506 | 2.023 | 80.726 |
| 6 | 2.398 | 1.964 | 81.902 |
| 7 | 2.533 | 1.941 | 76.629 |
| 8 | 2.348 | 2.102 | 89.523 |
| 9 | 2.493 | 1.875 | 75.211 |
| Average | 2.490 | 2.051 | 82.405 |
| SD | 0.094 | 0.129 | 4.580 |

Table 2 - Results for adhesive bandage with open cell (sponge) pad

| Measurements | Thickness (mm) | | Thickness recovery (%) |
|---|---|---|---|
| | Before pressure | After pressure | |
| 1 | 4.971 | 3.696 | 74.351 |
| 2 | 5.012 | 3.671 | 73.244 |
| 3 | 4.943 | 3.758 | 76.027 |
| 4 | 4.932 | 3.822 | 66.921 |
| 5 | 4.972 | 3.574 | 77.494 |
| 6 | 4.858 | 3.538 | 71.883 |
| 7 | 4.915 | 3.637 | 72.828 |
| 8 | 4.875 | 3.572 | 73.998 |
| 9 | 4.896 | 3.757 | 73.272 |
| Average | 4.9304 | 3.6694 | 73.34 |
| SD | 0.0499 | 0.0977 | 2.95 |

The table shows an adhesive bandage with a closed cell cushion pad had an average thickness recovery of 82.4% and the adhesive bandage with open cell (sponge) cushion pad presented an average thickness recovery of 73.3%, which is statically different ($p < 0.01$). The adhesive bandage with the closed cell pad presented a better thickness recovery than the adhesive bandage with the open cell (sponge) pad.

**Claims**

1. An adhesive bandage for use on wounds placed under compressive force, comprising:

   a support layer having a top side and an opposing bottom side,
   an adhesive layer deposited onto said bottom side of said support layer,
   a cushion pad associated with said adhesive layer, said cushion pad being substantially sealed,

an absorbent pad bonded to said cushion pad.

2. The adhesive bandage of claim 1 wherein said cushion pad provides an average percent thickness recovery of said cushion pad of at least 80% when said cushion pad is subjected to a force of 8N for five minutes.

3. The adhesive bandage of claim 1 or claim 2 wherein said absorbent pad comprises a hydrocolloid.

4. The adhesive bandage of claim 3 wherein said hydrocolloid is selected from the group consisting of guar gums, pectin, xanthan gum, gelatins, carboxymethylcellulose, sodium alginate, calcium alginates and polysaccharides.

5. The adhesive bandage of any preceding claim wherein said adhesive layer comprises a pressure-sensitive adhesive.

6. The adhesive bandage of any preceding claim wherein said absorbent pad comprises an active ingredient.

7. The adhesive bandage of any preceding claim wherein said active ingredient is selected from the group consisting of antibiotics, analgesics, antipyretics, antimicrobials, antiseptics, antiallergics, anti-acne, anesthetics, anti-inflammatories, hemostats, cosmetics, vitamins, vasodilators, emollients, pH regulators, antipruritics, counterirritants, antihistamines and steroids.

8. The adhesive bandage of any preceding claim wherein said cushion pad comprises a foam containing closed pores.

9. The adhesive bandage of claim 8 wherein the volume of said closed pores comprises from about 80 to about 99 percent of the volume of said cushion pad.

10. The adhesive bandage of claim 9 wherein the average diameter of said closed pores is about 0.1 mm to about 1.0mm.

**Fig. 1**

**Fig. 2**

50

58    52

56c

56a

54

**Fig. 3**

60

66c  68    62    66a

C ———————————— C

64

**Fig. 4a**

66b    68    66a

66c

66d    69

**Fig. 4b**

110

142    144    140    142

120    150

**Fig. 5**

Europäisches
Patentamt

European
Patent Office

Office européen
des brevets

# EUROPEAN SEARCH REPORT

**Application Number**

EP 10 25 1650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 203 21 148 U1 (RUESTER STEFAN [DE]) 9 March 2006 (2006-03-09) * paragraph [0041] - paragraph [0043]; figure 1 * ----- | 1,2 | INV. A61F13/02 |
| X | GB 2 387 331 A (JOHNSON & JOHNSON MEDICAL LTD [GB]) 15 October 2003 (2003-10-15) * figures 3,4 * * page 4, line 18 * * page 7, line 1 - page 7, line 17 * * page 8, line 22 - page 8, line 27 * * page 9, line 6 - page 9, line 22 * * claim 11 * ----- | 1-10 | |
| X | US 6 384 294 B1 (LEVIN JOHN M [US]) 7 May 2002 (2002-05-07) * figures 1,2,5 * * page 4, line 41 - page 4, line 64 * ----- | 1 | |
| X | EP 0 457 413 A1 (UTERMOEHLEN NV [NL]) 21 November 1991 (1991-11-21) * page 1, line 5 - line 13; figure 1 * ----- | 1-3 | TECHNICAL FIELDS SEARCHED (IPC) A61F |

The present search report has been drawn up for all claims

| Place of search | Date of completion of the search | Examiner |
|---|---|---|
| Munich | 1 February 2011 | Gennari, Silvia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 10 25 1650

This annex lists the patent family members relating to the patent documents cited in the above-mentioned European search report.
The members are as contained in the European Patent Office EDP file on
The European Patent Office is in no way liable for these particulars which are merely given for the purpose of information.

01-02-2011

| Patent document cited in search report | | Publication date | Patent family member(s) | | Publication date |
|---|---|---|---|---|---|
| DE 20321148 | U1 | 09-03-2006 | NONE | | |
| GB 2387331 | A | 15-10-2003 | AU<br>EP<br>WO | 2003229902 A1<br>1496826 A1<br>03086255 A1 | 27-10-2003<br>19-01-2005<br>23-10-2003 |
| US 6384294 | B1 | 07-05-2002 | NONE | | |
| EP 0457413 | A1 | 21-11-1991 | AT<br>CA<br>DE<br>DE<br>NL | 113193 T<br>2042839 A1<br>69104769 D1<br>69104769 T2<br>9001156 A | 15-11-1994<br>18-11-1991<br>01-12-1994<br>13-04-1995<br>16-12-1991 |

EPO FORM P0459

For more details about this annex : see Official Journal of the European Patent Office, No. 12/82